# EUROPEAN PATENT SPECIFICATION

(11) **EP 1 628 998 B1**
(45) Date of publication and mention of the grant of the patent: **23.02.2011**
(21) Application number: 04735606.8
(22) Date of filing: 01.06.2004
(51) Int. Cl.: C07K 1/13, C07K 1/16

(54) **A TAG FOR PURIFICATION OF PEPTIDES**
EIN TAG FÜR DIE REINIGUNG VON PEPTIDEN
MARQUEUR PERMETTANT DE PURIFIER DES PEPTIDES

(30) Priority: 30.05.2003 GB 0312426
(43) Date of publication of application: 01.03.2006
(73) Proprietor: Almac Sciences (Scotland) Limited, Edinburgh EH1 2EN (GB)
(72) Inventor: HAY, Alastair, Edinburgh EH12 8BZ (GB); COTTON, Graham, Edinburgh EH13 9PH (GB); RAMAGE, Robert, Edinburgh EH4 3JZ (GB)
(74) Representative: Main, Malcolm Charles
(86) International application number: PCT/GB2004/002327
(87) International publication number: WO 2004/106363

(56) References cited:
- EP-A1- 0 309 839
- EP-A2- 0 312 922
- WO-A-91/18596
- CANNE L E ET AL: "Synthesis of a Versatile Purification Handle for Use with Boc Chemistry Solid Phase Peptide Synthesis" TETRAHEDRON LETTERS, ELSEVIER SCIENCE PUBLISHERS, AMSTERDAM, NL, vol. 38, no. 19, 12 May 1997 (1997-05-12), pages 3361-3364, XP004061426 ISSN: 0040-4039
- BROWN ANGUS R ET AL: "Affinity purification of synthetic peptides and proteins on porous graphitised carbon" TETRAHEDRON LETTERS, vol. 34, no. 44, 1993, pages 7129-7132, XP002299070 ISSN: 0040-4039
- VIZZAVONA J ET AL: "Covalent capture purification of polypeptides after SPPS via a linker removable under very mild conditions" TETRAHEDRON LETTERS, ELSEVIER SCIENCE PUBLISHERS, AMSTERDAM, NL, vol. 43, no. 48, 25 November 2002 (2002-11-25), pages 8693-8696, XP004390463 ISSN: 0040-4039
- BALL H L ET AL: "PURIFICATION OF SYNTHETIC PEPTIDES USING REVERSIBLE CHROMATOGRAPHIC PROBES BASED ON THE FMOC MOLECULE" INTERNATIONAL JOURNAL OF PEPTIDE AND PROTEIN RESEARCH, MUNKSGAARD, COPENHAGEN, DK, vol. 40, no. 5, 1 November 1992 (1992-11-01), pages 370-379, XP000316564 ISSN: 0367-8377
- BALL H L ET AL: "Purification of synthetic peptides with the aid of reversible chromatographic probes" JOURNAL OF CHROMATOGRAPHY A, vol. 686, no. 1, 1994, pages 73-83, XP004023391 ISSN: 0021-9673
- BALL H L ET AL: "Affinity purification of 101 residue rat cpn10 using a reversible biotinylated probe." JOURNAL OF PEPTIDE SCIENCE : AN OFFICIAL PUBLICATION OF THE EUROPEAN PEPTIDE SOCIETY. 1995 SEP-OCT, vol. 1, no. 5, September 1995 (1995-09), pages 288-294, XP002299071 ISSN: 1075-2617
- KAZMIERSKI W M ET AL: "Synthesis of the Carbonic Acid Benzotriazol-1-yl-Ester-(2-Biotinylami no)-9H-Fluoren-9-ylmethyl Ester: A Convenient Transient-Biotinylation Reagent for Use in Affinity Chromatography" TETRAHEDRON LETTERS, ELSEVIER SCIENCE PUBLISHERS, AMSTERDAM, NL, vol. 36, no. 50, 11 December 1995 (1995-12-11), pages 9097-9100, XP004026691 ISSN: 0040-4039
- KELLAM B ET AL: "Transient Affinity Tags Based on the Dde Protection/Deprotection Strategy: Synthesis and Application of 2-Biotinyl- and 2-Hexanoyldimedone" TETRAHEDRON LETTERS, ELSEVIER SCIENCE PUBLISHERS, AMSTERDAM, NL, vol. 38, no. 30, 28 July 1997 (1997-07-28), pages 5391-5394, XP004083327 ISSN: 0040-4039
- RAMAGE R ET AL: "DESIGN OF AN AFFINITY-BASED N-ALPHA AMINO PROTECTING GROUP FOR PEPTIDE SYNTHESIS TETRABENZO-A C G I-FLUORENYL-17-METHYL URETHANES TBFMOC" TETRAHEDRON LETTERS, vol. 33, no. 3, 1992, pages 385-388, XP001183595 ISSN: 0040-4039
- MERRIFIELD R B ET AL: "9-2 SULFOFLUORENYLMETHYLOXY CARBON CHLORIDE A NEW REAGENT FOR THE PURIFICATION OF SYNTHETIC PEPTIDES" JOURNAL OF ORGANIC CHEMISTRY, vol. 43, no. 25, 1978, pages 4808-4816, XP002314808 ISSN: 0022-3263
- YING-ZENG LIU ET AL: "A NOVEL FMOC-BASED ANCHORAGE FOR THE SYNTHESIS OF PROTECTED PEPTIDES ON SOLID PHASE" INTERNATIONAL JOURNAL OF PEPTIDE AND PROTEIN RESEARCH, MUNKSGAARD, COPENHAGEN, DK, vol. 35, no. 2, 1 February 1990 (1990-02-01), pages 95-98, XP000085133 ISSN: 0367-8377
- ZIER A ET AL: "POLYETHYLENE GLYCOL BOUND BENZYL- AND FLUORENYL DERIVATIVES AS SOLUBILIZING SIDE-CHAIN PROTECTING GROUPS IN PEPTIDE SYNTHESIS" TETRAHEDRON LETTERS, ELSEVIER SCIENCE PUBLISHERS, AMSTERDAM, NL, vol. 35, no. 7, 1994, pages 1039-1042, XP002950143 ISSN: 0040-4039
- UEDA E K M ET AL: "Current and prospective applications of metal ion-protein binding" JOURNAL OF CHROMATOGRAPHY A, ELSEVIER SCIENCE, NL, vol. 988, no. 1, 21 February 2003 (2003-02-21), pages 1-23, XP004405859 ISSN: 0021-9673 cited in the application

## Description

### Field of the Invention

The present invention relates to purification means, in particular to means suitable for use in purification of synthetic peptides and proteins.

### Background to the Invention

There are few examples of chemical tags which have been used to enhance the properties of chemically synthesised peptides and proteins. Ramage et al (R. Ramage and G. Raphy, Tetrahedron Lett., 1992, 33, 385-388) used Tbfmoc as a hydrophobic analogue to Fmoc for the purification of peptides and proteins. Tbfmoc was found to be extremely hydrophobic, and has sufficient effect on the elution of peptides and proteins with the tag appended, to assist in preparative HPLC purification. The affinity of charcoal for porous graphitised carbon (PGC) has also been used for the chromatographic purification of peptides and proteins. However, the Tbfmoc group was found to have such a high affinity for hydrophobic surfaces that undesired binding to surfaces was often unavoidable. Furthermore, Tbfmoc has an unfavourable effect on the solubility of peptides and proteins in aqueous systems, often resulting in difficulties during purification.

The inherent affinity of some amino acids, such as histidine (His), to metal ions has been utilised in the purification of some peptides and proteins. One technique involves adding a poly-His (E. Hendan and J. Porath, J. Chromatography, 1985, 323, 255-264) sequence to the terminus of the peptide or protein sequence of interest, and using the affinity of the appendage for nickel ions to selectively bind the desired product to a surface. Although effective, the poly-His tag remains a permanent feature of the parent sequence, which may affect folding, hence activity. Moreover, the extra synthetic steps to add the tag may reduce overall yield of material.

A cleavable poly-His tag has been reported (Servion et al, EP 0827966), in which a methionine residue is inserted between the parent peptide sequence and the histidine residues. Cleavage at methionine residues is achieved selectively by treatment with cyanogen bromide. However, cyanogen bromide is a highly toxic reagent, and its use in such conditions is not robust. Furthermore, methionine residues in the protein sequence are used in the oxidised form to prevent undesired cyanogen bromide cleavage. These methionine residues must then be reduced to obtain the native sequence.

The natural electron donating properties of some amino acids to enable ligation to metal centres has also been used to purify recombinant proteins (H. Chaouk, M. T. W. Hearn, J. Chromatography A, 1999, 852, 105-115).

Tags that covalently bind to a functionalized solid support have been used to purify both synthetic and recombinant proteins (M. Villain, J. Vizzavona, and K. Rose, Chemistry & Biology, 2001, 8, 673-679; J Vizzavona, M. Villain, and K. Rose, Tetrahedron Lett, 2002, 8693-8696). Initially, the method was applicable only to proteins with N-terminal cysteine or threonine, but the method was tailored to be suitable for all N-terminal amino acids (J Vizzavona, M. Villain, and K. Rose, Tetrahedron, Lett, 2002, 8693-8696). However, obtaining the chemical tag requires a lengthy synthesis, and is cleaved under conditions (sodium periodate) that may damage the peptide or proteins.

Canne et al (1997), Tetrahedron Lett., 38.3361-3364, describes a Boc derived removable handle coupled to the NH₂ terminus of a synthesised peptide for use in a purification method for peptides.

Ball et al (1994), J. Chromatography A., 686, 73-83 describes purification of synthetic peptides with the aid of reversible chromatography probes based on Fmoc and also describes the use of a probe containing biotin for affinity chromatography.

Kellan et al (1997) Tetrahedron Lett., 38,5391-5394 describes transient affinity tags based on Dde group.

A much sought after aspect of proteomics is the generation of arrays of peptides or proteins on surfaces, for example polystyrene multi-well plates. One method of achieving oriented peptide arrays uses anthraquinones, which bind to polymers following uv irradiation (S. P. Jensen, S. E. Rasmussen, M. H. Jakobsen, Innovations and Perspectives in Solid Phase Synthesis & Combinatorial Chemical Libraries, 1996, 419-422). The anthraquinone is functionalised in such away as to enable amide coupling to the N-terminus of a peptide, hence the peptide becomes covalently bound to the polystyrene surface.

There therefore remains a need for chemical tags which can be used for purification of a peptide and which may be efficiently and easily cleaved from the peptide whilst minimising damage to the peptide itself.

### Summary of the Invention

The present inventors have developed a tag which overcomes many of the problems associated with the tags of the prior art.

According to a first aspect of the present invention, there is provided a tag for purification of peptides, said tag molecule having structural formula I:

(A)ₙ-C Formula I

wherein A is a capture moiety,
n is at least 1, e.g. 1, 2, 3 or 4; and
C is a peptide binding moiety having formula II: 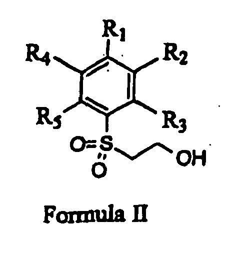 wherein,
R₁ and R₃ are each independently H or any C₁₋₆ alkyl group or an electron withdrawing group, R₂ is H or any C₁₋₆ alkyl group, one of R₄ and R₅ is a linker moiety B which links said binding moiety to A; R₄, when not the linker moiety B, is H or any C₁₋₆ alkyl group; Rₛ, when not the linker moiety B, is an electron withdrawing group or H or any C₁₋₆ alkyl group; wherein at least one of R₁, R₃ and R₅ is an electron withdrawing group;

Alternatively or in addition, where C is the peptide binding moiety having Formula II, one or more of R₂, R₃, R₄ (when not the linker moiety B) and R₅ (when not the linker moiety B) may be each independently any other substituent which does not significantly affect the stability of the tag when used to tag a peptide. Such substituents preferably have no or only mild electronic effects, for example amide or aryl groups. However, substituents with more pronounced electronic effects which nevertheless do not prevent the tag binding with a peptide may be used. Such substituents may include halogen, nitro, ester, alkoxy or aldehyde group(s).

In preferred embodiments of the invention, whereby C is the peptide binding moiety having Formula II, only one of R₁, R₃ and R₅ is an electron withdrawing group. In a preferred embodiment, R₁ is an electron withdrawing group, R₃ is H or any C₁₋₆ alkyl group and R₅ is the linker moiety B, H or any C₁₋₆ alkyl group.

The invention further provides a tag molecule as claimed in claim 28.

The electron withdrawing group (s) of Formula II may be any electron withdrawing group, preferably an electron withdrawing group selected from the group comprising I, Br, Cl, SO₂CH₃, CF₃, and NO₂. In a particularly preferred embodiment, the electron withdrawing group (s) is NO₂.

This arrangement facilitates the easy cleavage of a bond formed between the tag molecule and a peptide to which it is bound.

The capture moiety may comprise any group suitable for binding of the tag molecule to another molecule "the capture receptor", and which may be used to separate the tag molecule from other molecules not comprising the capture moiety.

In a preferred embodiment, the capture molecule is hydrophobic and may comprise one or more hydrophobic groups. The hydrophobic group may be substituted or unsubstituted. Suitable subtituents may include but are not limited to alkyl, alkoxy, amino or halo groups. In preferred embodiments, the hydrophobic group is unsubstituted. In one embodiment, the capture moiety comprises the ring structure shown as formula III, 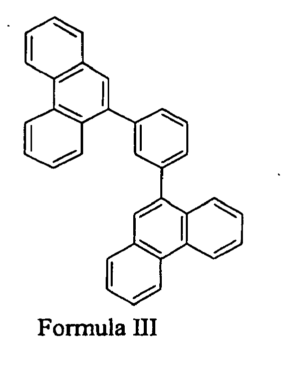 wherein said binding moiety is linked to the linker moiety at position 5 of the central phenyl group of formula III. In a preferred embodiment, the tag molecule of the invention has formula IV:

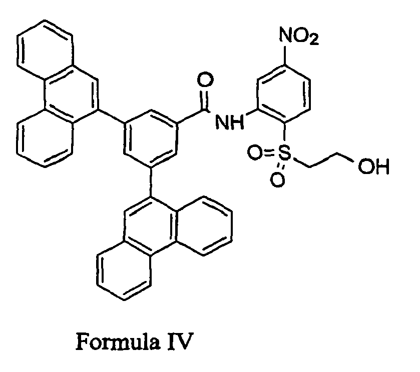

In a particularly preferred embodiment in which the capture moiety comprises a hydrophobic group, the capture moiety comprises the ring structure as shown below as Formula IIIA or, the ring structure as shown below as Formula IIIB. In embodiments where the capture moiety comprises the ring structure as shown as Formula IIIA or Formula IIIB, the tag molecule of the invention has formula IVA or, more preferably, IVB respectively as shown below.

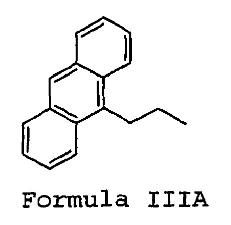

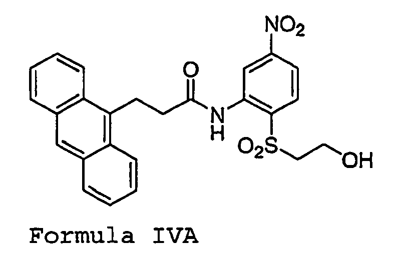

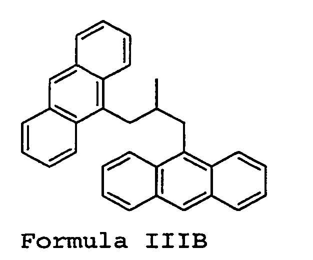

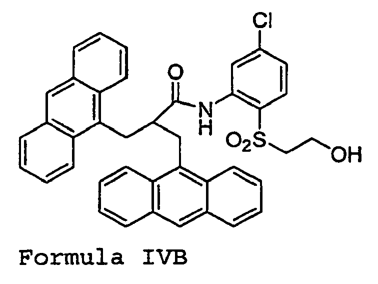

As with the capture moiety having Formula III, the carbon atoms may each independently be substituted or unsubstituted. Suitable substituents may include but are not limited to alkyl, alkoxy, amino or halo groups. In preferred embodiments, the carbon atoms of the capture moieties having formula IIIA or IIIB is unsubstituted. In particularly preferred embodiments of the invention, the capture moiety has Formula III, Formula IIIA or Formula IIIB.

In some alternative preferred embodiments, the capture moiety A may be a metal-binding moiety, which may be substituted or unsubstituted. If present, substituent (s) are preferably selected such that they do not reduce or do not significantly reduce the degree of binding of the moiety to a metal ion compared to the degree of binding by a corresponding moiety lacking the substituent(s). Suitable substituents may include but are not limited to alkyl, alkoxy, amino or halo groups. Preferably, the moiety is unsubstituted.

Suitable metal-binding moieties may include, but are not limited to, pyridyl groups, hexa-histidine, iminodiacetic acid (IDA), nitrilotriacetic acid (NTA), 8-hydroxyquinoline and O-phosphoserine (Ueda, et al, J. Chromatography A, 2003, 988, 1-23. In one preferred embodiment, the metal binding moiety is a hexa-histidine tag. In another preferred embodiment, the metal binding moiety is a pyridyl group. Preferably, the pyridyl group is unsubstituted. In a particularly preferred embodiment, the capture moiety has formula V:

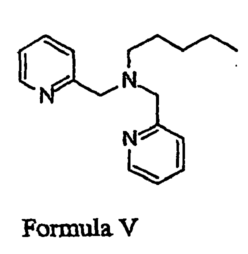

A particularly preferred tag of the invention having a metal binding capture moiety has formula VI:

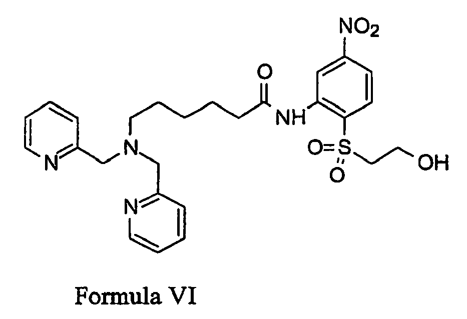

The tag molecule of the invention may be used to tag any peptide, polypeptide or protein. In this application, unless the context demands otherwise, the terms peptide, polypeptide and protein are used interchangeably.

Tagged peptides comprising a tag molecule according to the invention attached to a peptide constitute a second aspect of the present invention. Preferably the peptide is attached to the tag molecule through a carbamate bond.

According to a third aspect of the present invention, there is provided a method of tagging a peptide molecule, said method comprising providing a tag molecule according to the first aspect of the invention wherein the hydroxy group of formula II is substituted with a reactive moiety, and reacting the substituted tag molecule with a peptide molecule under suitable reaction conditions, preferably basic conditions, wherein said reactive moiety is a moiety having formula VII: 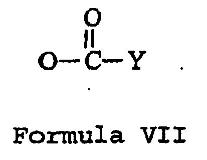 wherein Y is any halogen, preferably Cl, or said reactive moiety comprises a carbonyldioxy moiety.

The substituted tag molecule may be formed using any suitable reaction. For example, in preferred embodiments, the substituted tag molecule may be formed by reacting the tag molecule according to the first aspect of the invention with phosgene, bis(4-nitrophenyl)carbonate, bis(pentafluorophenyl) carbonate or N,N'-di-succinimidyl carbonate under suitable conditions, e.g. basic conditions.

The tag molecules of the invention may be used to separate and/or purify a peptide from a mixture of peptides and/or other components. Accordingly, in a fourth aspect, the invention provides a method for the modification of peptides for facilitating purification thereof, the method comprising the step of: attaching a tag molecule according to the first aspect of the invention to the end of a peptide chain during synthesis thereof.

In an alternative embodiment, the tag of the invention may be coupled to a single amino acid. This tagged amino acid can be used as an N-terininal. amino acid which can be coupled using standard peptide synthetic methods to the remainder of a peptide sequence. Such a tagged amino acid can be prepared by, e.g. providing a tag molecule according to the first aspect of the invention wherein the hydroxy group of formula II is substituted with a reactive moiety, and reacting the substituted tag molecule with an amino acid (possibly with side chain, or carboxy protection) under suitable reaction conditions (preferably basic conditions) wherein said reactive moiety is a moiety having formula VII: 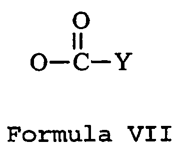 wherein Y is any halogen, preferably Cl, or said reactive moiety comprises a carbonyldioxy moiety.

The tagged amino acid may then be coupled to the remainder of the peptide sequence using standard peptide coupling methods.

Thus, in a fifth aspect of the present invention, there is provided a tagged amino acid comprising a tag molecule according to the first aspect of the invention attached to an amino acid. Preferably the amino acid is attached to the tag molecule through a carbamate bond.

An example of carbamate bond is shown below as Formula VIII:

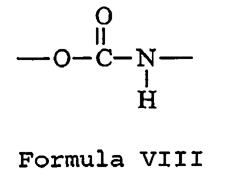

According to a sixth aspect of the present invention, there is provided a method of tagging an amino acid comprising providing a tag molecule according to the first aspect of the invention wherein the hydroxy group of formula II is substituted with a reactive moiety, and reacting the substituted tag molecule with an amino acid molecule under suitable reaction conditions, preferably basic conditions. The reactive moiety is a moiety having formula VII: 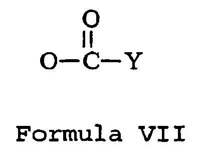 wherein Y is any halogen, preferably Cl, or the reactive moiety comprises a carbonyldioxy moiety. The method optionally comprises the further step of coupling the amino acid to a further amino acid or peptide molecule.

In a seventh aspect, the invention provides a method for the modification of a peptide for facilitating purification thereof, comprising the steps:
a) attaching a tag molecule according to the first aspect of the invention to the N-terminus of an amino acid, and
b) coupling the tagged amino acid formed in step (a) to the peptide.

According to an eighth aspect of the present invention there is provided a method of purifying a peptide comprising:
a) providing a sample comprising a tagged peptide according to the second aspect of the invention or prepared according to the third, sixth or seventh aspect of the invention,
b) bringing said sample into contact with a capture receptor with which the capture moiety has affinity,
c) removing unbound molecules, for example, by washing,
d) optionally removing bound tagged peptide from the capture receptor, and
e) cleaving the peptide from the binding moiety of the tag molecule.

Any suitable capture receptor may be used. For example, the capture receptor may be provided as part of an HPLC column.

As described above, the tag molecule of the present invention is particularly advantageous in that a peptide tagged with said molecule can easily be cleaved from the tag molecule following purification under conditions which minimise, indeed preferably prevent, damage to the peptide. In particularly preferred embodiments of the method of the eighth aspect of the invention, in step e) said peptide is cleaved from said binding moiety under basic conditions.

Biological assays and diagnostic tests commonly involve the binding of peptides or proteins to surfaces such as well surfaces of multi-well plates. However, the binding of such peptides or proteins, either directly or via conventionally used tag molecules, often results in the peptides being randomly oriented on the well surface, giving results with a high background noise and poor reproducibility.

It is believed that the tag molecules of the invention are particularly efficient at securely anchoring the tag molecules to the surface and allowing the peptide or protein to become uniformly orientated, thus ensuring that substantially all of the peptide or protein is available for molecular binding to a substrate. This uniformity increases the reliability and reproducibility of a diagnostic test.

Therefore, according to a ninth aspect of the present invention, there is provided a method of spatially orientating peptides on a surface in a substantially uniform direction comprising the steps of:
a) providing tagged peptides according to the second aspect of the invention or prepared according to the third, sixth or seventh aspect of the invention,
b) bringing the tagged peptides into contact with a capture receptor with which the capture moiety has affinity,
c) allowing the tagged peptides to bind to the surface via interaction of the capture moiety with the capture receptor.

In a further aspect of the invention, there is provided a diagnostic kit for the detection or purification of a peptide, said kit comprising:
a) a molecular tag according to the first aspect of the present invention or a tagged amino acid according to the fifth aspect of the invention, and
b) a capture receptor which can bind the capture moiety of the molecular tag.

### Capture Moiety

Capture moieties may include any suitable ligand which binds to a corresponding ligand-binding molecule. For the purposes of the present application, unless the context demands otherwise, the ligands may include, but are not limited to, haptens, antibodies and lipophilic molecules. Preferably, the ligand and corresponding ligand binding molecule have binding specificity for one another. The members of such a binding pair may be naturally derived or wholly or partially synthetically produced. One member of the pair of molecules may have an area on its surface, which may be a protrusion or a cavity, which specifically binds to and is therefore complementary to a particular spatial and polar organisation of the other member of the pair of molecules. Thus, the members of the pair may have the property of binding specifically to each other.

For the purposes of the present application, unless the context demands otherwise, references to ligands may also be taken to refer to, but are not limited to, haptens, antibodies and lipophilic molecules. Thus the capture moiety may be a hapten which binds to a hapten binding molecule, for example an antibody. Any suitable hapten for which a specific antibody is known or can be generated may be used as a capture moiety. An example of a suitable hapten is digoxigenin (Kerkhof, Anal. Biochem. 205:359-364 (1992).

In another embodiment, antibodies can be used as the capture moiety with suitable haptens used to capture the tags. In one embodiment, the capture moiety and the hapten to which it binds may be antibodies. For example, the capture moiety may be an antibody with the hapten to which it binds being an anti-antibody, for example an antibody directed against an antibody of a certain species or directed against a specific class of antibodies e.g. IgG, IgM.

An "antibody" is an immunoglobulin, whether natural or partly or wholly synthetically produced. The term also covers any polypeptide, protein or peptide having a binding domain which is, or is homologous to, an antibody binding domain. These can be derived from natural sources, or they may be partly or wholly synthetically produced. Examples of antibodies are the immunoglobulin isotypes and their isotypic subclasses and fragments which comprise an antigen binding domain such as Fab, scFv, Fv, dAb, Fd; and diabodies. Methods to produce such antibodies and fragments are well known in the art.

In a further embodiment, biotin may be used as the capture moiety with streptavidin or biotin specific antibodies used as the capture receptor to capture the tag molecule.

In yet another embodiment of the present invention, the capture moiety is a lipophilic molecule. The use of such molecules as the capture moiety may enable separation of a tagged peptide from contaminating non-tagged molecules in a mixture or composition using, for example, reverse HPLC (High Performance Liquid Chromatography). Examples of lipophilic molecules which can be used as capture moieties in the tag molecules or the methods of the invention include, but are not limited to, tocopheryl, cholesteryl and long chain alkyl groups.

In one preferred embodiment, the capture moiety has formula III 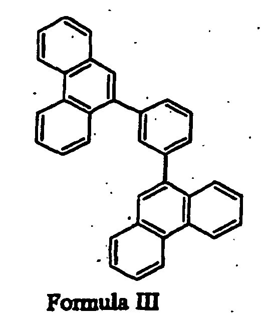 wherein said binding moiety is linked to the linker moiety at position 5 of the central phenyl group of the structure having formula III.

In another preferred embodiment, the capture moiety comprises the ring structure as shown below as Formula IIIA or, the ring structure as shown below as Formula IIIB:

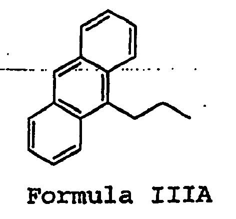

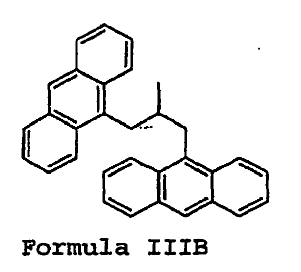

Where the tag is designed for binding to a metallic centre or metal containing surface such as Zn²⁺, Co²⁺, Cu²⁺ or Ni²⁺, the capture moiety may be a metal-binding moiety, for example, one or more pyridyl groups, poly-histidine, iminodiacetic acid (IDA), nitrilotriacetic acid (NTA), 8-hydroxyquinoline or O-phosphoserine groups. In a particularly preferred embodiment, the capture moiety has formula V

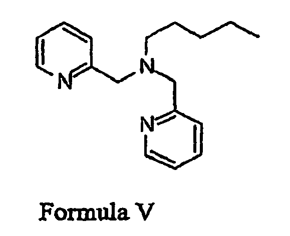

### Capture Receptors

The choice of capture moiety will be determined by the skilled person based on the capture receptor with which the tag molecule is envisioned for use. Capture receptors encompass any molecule, structure or surface with which the capture moiety can interact and which can be used to separate the tagged peptide from other non-tagged peptides.

The capture receptor may be or may be associated with, for example bound to, coupled to or mounted on, any molecule, support or surface which enables capture of tagged molecules. Such molecules, supports or surfaces may be in the form of microplates, including multi-well plates, beads, membranes, bottles, dishes, microscope slides, fibers, polymers, particles and microparticles etc. They may be of any suitable material, including, but not limited to, acrylamide, cellulose, collagen, nitrocellulose, glass, polymers such as polystyrene, polyethylene, polypropylene, polysilicate, polycarbonate, teflon, polyamino acid, magnetic and /or metallic materials.

The nature of the capture receptor and the material of which it is constructed or on which it may be associated or mounted will be chosen by the skilled person depending on the tag molecule and the proposed use. For example, in some preferred embodiments, the capture receptor is provided as or on a solid support from which unbound molecules can be easily washed off thus enabling easy purification of tagged molecules from a mixture.

For example, the capture receptor may be a surface to which the tag of the invention covalently binds.

Capture receptors can be bound to a support using established coupling methods. Attachment agents and methods suitable for use in attaching capture receptors to solid supports are well known in the art and are described in, for example, Protein Immobilization: Fundamentals and Applications, Richard F. Taylor, ed. (M. Dekker, New York, 1991) and Immobilized Affinity Ligands, Craig T. Hermanson et al., eds. (Academic Press, New York, 1992).

For example, where the capture receptor is an antibody, immobilization of the antibody can be accomplished by attachment, for example, to aminated surfaces, carboxylated surfaces or hydroxylated surfaces using standard immobilization chemistries.

Additionally, many protein and antibody columns are available commercially as are reverse phase HPLC columns based on polystyrene.

### Removal of Peptides From Capture Tag

A particular advantage of the tag molecule of the present invention is that a peptide bound to the tag molecule may be easily cleaved without damage to the peptide, allowing easy purification and recovery of a the peptide. Typically, cleavage may be achieved using basic conditions such as 20% piperidine, in aqueous acetonitrile or 20% piperidine and 1% DBU, in aqueous acetonitrile.

Preferred features of each aspect of the invention are as for each of the other aspects mutatis mutandis.

The invention will now be described further in the following non-limiting examples with reference made to the accompanying drawings in which:
Figure 1 illustrates a route of synthesis of a metal binding tag molecule of the invention.
Figure 2A illustrates a route of binding of the metal binding tag of the invention to a peptide.
Figure 2B shows an HPLC trace of a cleavage reaction on a purified tagged peptide.
Figure 2C illustrates DPHSA-tagged peptide showing binding to Ni²⁺ charged HiTrap chelation column (Amersham Biosciences).
Figure 3 illustrates an alternative route of synthesis of a metal binding tag molecule of the invention and of binding the tag molecule to a peptide.
Figure 4 illustrates a route of synthesis of a hexa-histidine tagged glycine.
Figure 5 illustrates synthesis of histidine-tagged hydroxyl sulphide.
Figure 6 illustrates a route of synthesis of a hydrophobic tag molecule of the invention.
Figure 7A illustrates a route of synthesis of another hydrophobic tag molecule of the invention.
Figure 7B shows an HPLC Trace of Substance P(1), IIIA-appended Substance P (2), and IIIB-appended Substance P (3)
Figure 8 illustrates a synthetic route to a hexa-histidine tagged fluorenyl methanol tag of the invention

### Example 1 Synthesis of Metal Binding Tag

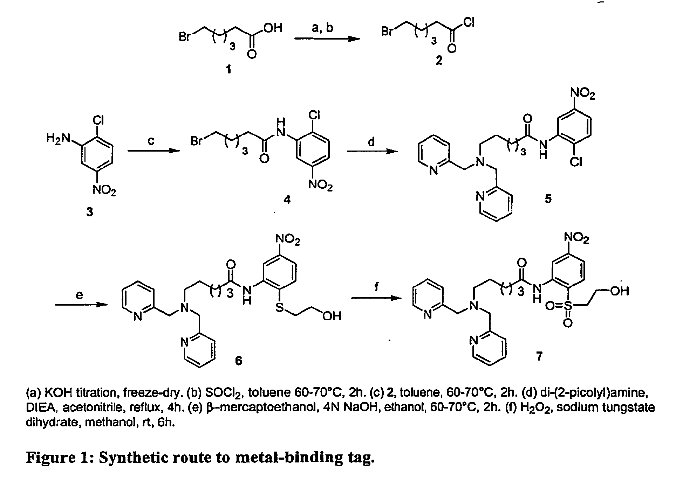

The synthesis of 6-(di-(2-picolyl)amine)-hexanoyl (2-(2-hydroxy-ethanesulfonyl)-5-nitro-phenyl]-anilide [DPHSA] is illustrated in Figure 1. Briefly, The tag, DPHSA is prepared in 6 steps from 6-bromohexanoic acid, by first forming the potassium salt following titration with aqueous KOH. The freeze-dried salt was then reacted with thionyl chloride to form the acid chloride, 2, which was reacted *in situ* in hot toluene with 2-chloro-5-nitroaniline to form bromoanilide 4. Direct Amide formation via the activated ester of carboxylic acids (*e.g*. treatment with DIC, HOBt) was found to be unsuccessful. Alkylation of di-(2-picolyl)amine with **4** was achieved by refluxing with DIEA in acetonitrile. Use of other bases such as potassium carbonate, and sodium hydride yielded complex byproducts. Sulfanylation of 5 was achieved by reaction with β-mercaptoethanol, and selective oxidation to sulfone, 7, by mild reaction with aqueous hydrogen peroxide. Over-oxidation to pyridine *N*-oxides were avoided by portion-wise addition of H₂O₂, careful selection of catalyst, and room temperature conditions.

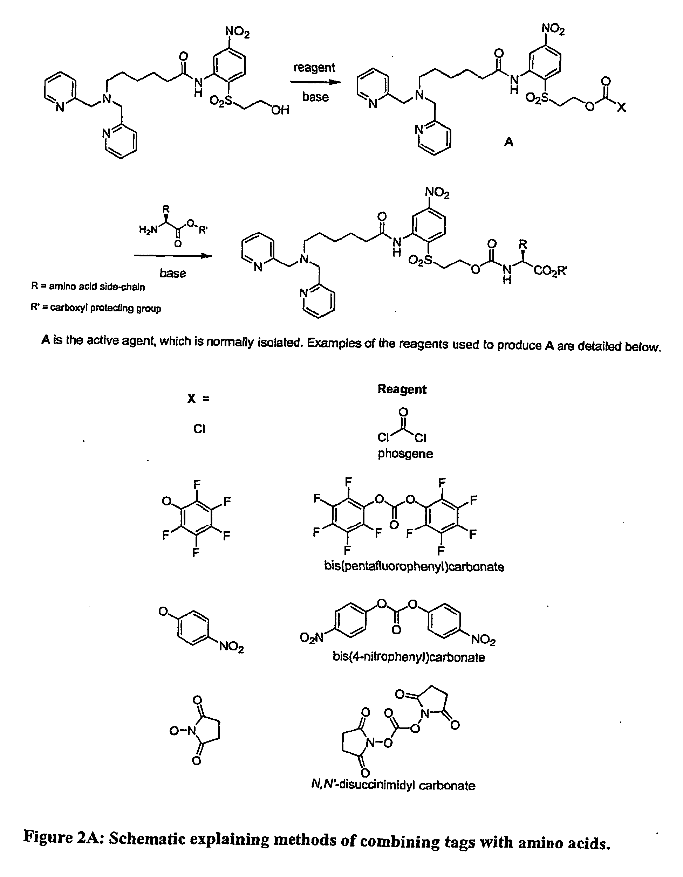

**Example 2 Purification of A Peptide using DPHSA** A route of synthesis of a peptide tagged with DHPSA is shown in Figure 2A.

DPHSA was reacted with an active material e.g. phosgene, bis(4-nitrophenyl)carbonate), bis(pentafluorophenyl)carbonate or N,N'-di-succinimidyl carbonate, under basic conditions to form a reactive moiety intermediate **A** in which, e.g where the active material is phosgene, X is Cl.

The peptide was prepared by standard solid or solution phase synthesis methods with the carboxylic group protected using a conventional protecting group.

The intermediate was then reacted with the peptide in the presence of an amine base (*e.g.* diisopropylethylamine).

In alternative embodiments, the reaction may involve a second intermediate. For example, the reactive moiety intermediate A in which X is Cl A may be constructed by reaction of the tag molecule with phosgene as described above. This may then be reacted with a second reactant e.g pentafluorophenol, *p*-nitrophenol,or di-N-hydroxy succinimide to form a second intermediate. The second intermediate is then reacted with the peptide in the presence of an amine base (*e.g*. diisopropylethylamine) as described above.

The tagged peptide is cleaved from the resin if required using standard cleavage conditions.

### Purification

The crude lyophilised peptide is dissolved in Buffer 1 (8M urea, 0.1M NaH₂PO₄, 0.01M Tris-Cl, pH 6.5) and agitated with capture resin (agarose, containing Ni²⁺ (or Zn²⁺) ligated with NTA).

The supernatant is removed and the resin is washed repeatedly with Buffer 2 (8M urea, 0.1M NaH₂PO₄, 0.01M Tris-Cl, pH 6.3).

The supernatant is removed and the protein is eluted with Buffer 3 (8M urea, 0.1M NaH₂PO₄, 0.01M Tris-Cl, pH 4.9).

The product solution is desalted by passing through a Sephadex column.

The tag is cleaved from the lyophilised product by treatment under basic conditions (*e.g*. 20% piperidine, in aqueous acetonitrile).

Although the above purification protocol could be used for purification of the tagged peptide, improved stability was obtained using the following protocol. Binding was checked using tagged material that had already been purified by HPLC.

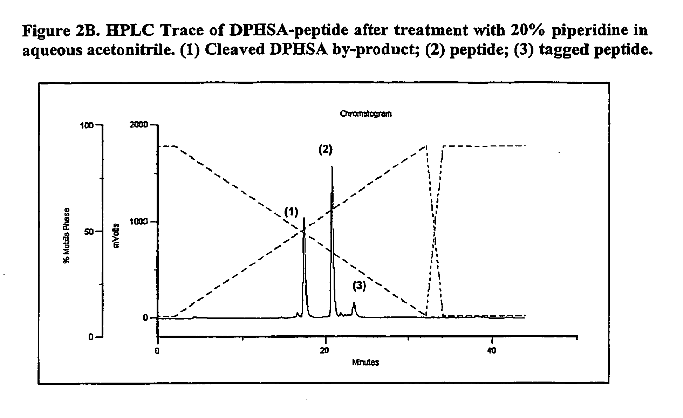

The purified lyophilised peptide was dissolved in buffer A (50mM sodium phosphate, 300mM, NaCl, pH 6.5) and shaken gently with capture resin (agarose, containing Ni²⁺, ligated with NTA). The supernatant was removed and the resin was suspended in Buffer A and transferred to a sintered gravity-flow column. The resin was washed with 5 column volumes of Buffer A, then product eluted with 10 column volumes of Buffer B (50mM sodium acetate, 300mM NaCl, pH 3.5), or a buffer with high imidazole concentration (50mM sodium phosphate, 300mM NaCl, 500mM imidazole). The peptide was then cleaved from the lyophilised product by treatment under alkaline conditions (*e.g*. 20% piperidine in aqueous acetonitrile). Figure 2B shows an HPLC trace of such a cleavage reaction on a purified tagged peptide.

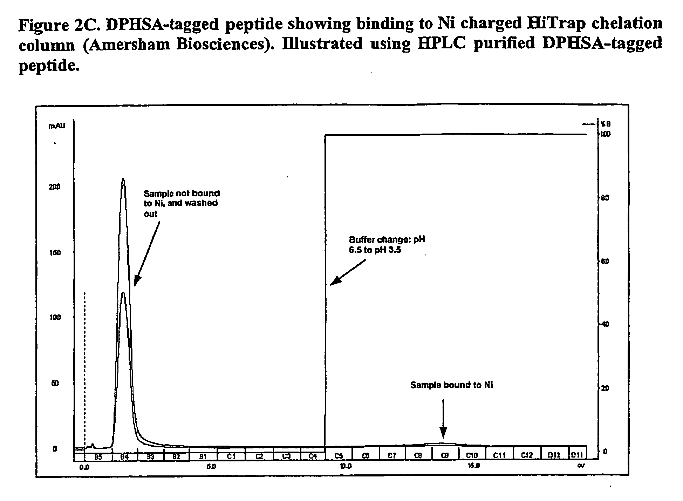

Preloaded Hi-Trap chelation columns (Amersham Biosciences) charged with Ni²⁺ can be used in a continuous flow manner, by charging sample in buffer (40mM sodium phosphate, 300mM NaCl, 8M urea, pH 6.5), and washing unbound sample with the same buffer. Bound sample can be removed from the nickel column by passing a buffer at lower pH (40mM sodium phosphate, 300mM NaCl, 8M urea pH 3.5). This was demonstrated using HPLC purified DPHSA-tagged peptide and is illustrated in Figure 2C, which shows DPHSA-tagged peptide showing binding to Ni²⁺ charged HiTrap chelation column (Amersham Biosciences).

Alternatively, the peptide could be cleaved (after removal of non-bound impurities by washing) whilst tagged to the resin under alkaline conditions (for example pH 9.0), and the free peptide desalted using a Sephadex column.

### Example 3 Synthesis of a Metal Binding Tag and Tagging to a Peptide

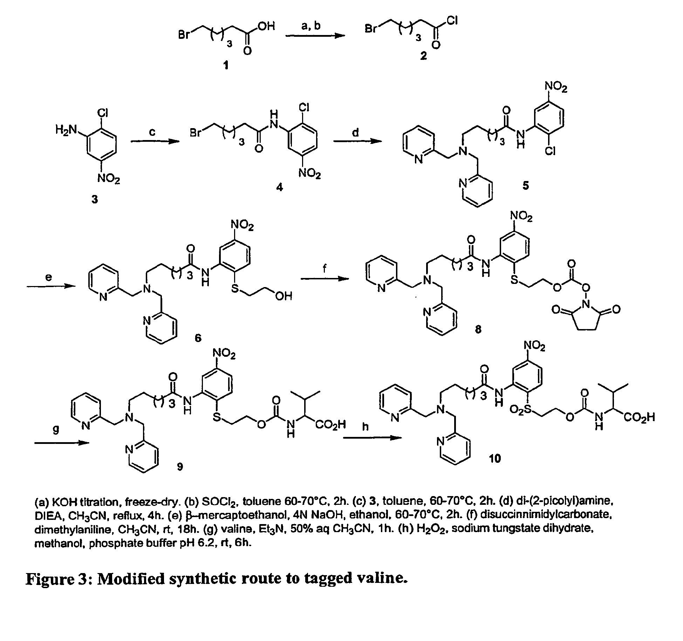

An alternative route to that described in Figures 1 and 2A which has been used in the preparation of a tagged amino acid using DPHSA is shown in Figure 3.

As can be seen in Figure 3, the synthetic route was identical to that illustrated in Figure 1 to the sulfanylated intermediate 6. Thereafter, instead of selective oxidation to the sulfone 7 as in Figure 1 prior to reaction with the active material (e.g. phosgene, N,N'di-succinimidyl carbonate to form intermediate 1, which is then reacted with the peptide as in Figure 2A, the sulfanylated intermediate 6 was reacted with the active material (e.g N,N'di-succinimidyl carbonate) to form intermediate 8 (Figure 3), which was then reacted with the amino acid (in the case of Figure 3, valine) to form intermediate 9 which was then selectively oxidised to the sulfone structure shown as 10 in Figure 3.

Although the method shown in Figure 3 shows tagging of valine, the method is applicable to tagging of other amino acids and peptides.

### Example 4 Synthesis of a Metal Binding Hexa-Histidine Tag Tagging to a Peptide

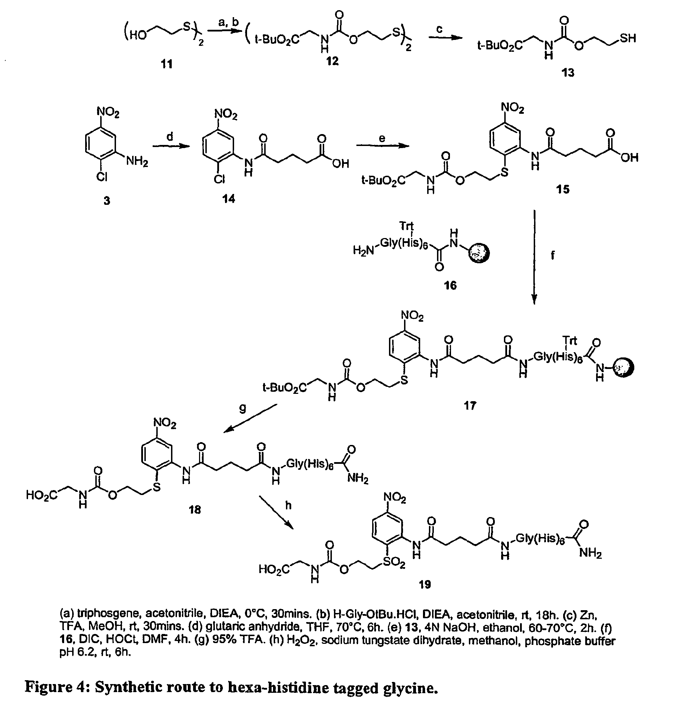

Figure 4 illustrates an aspect of the invention in which a hexa-histidine tag is used to tag an amino acid/peptide using glycine for exemplification. Standard solid phase peptide synthesis using Fmoc protocols is used to generate resin-bound hexa-histidine, with trityl protection on the side-chains, **16.** This is coupled with an N-terminally modified amino acid t-butyl ester (*e.g.* glycine, **15**). Treatment with 95% TFA affords cleavage of the tagged amino acid from the resin, as well as concomitant cleavage of side-chain trityl groups, and *t*-butyl ester to form **18** (which may be purified by HPLC if required). Oxidation in mildly acidic conditions affords the target molecule, **19.** The hexa-histidine-glycine tag is then coupled directly to the *N*-terminus of a peptide or protein by standard amide coupling methods (*e.g.* DIC, HOCt, DMF).

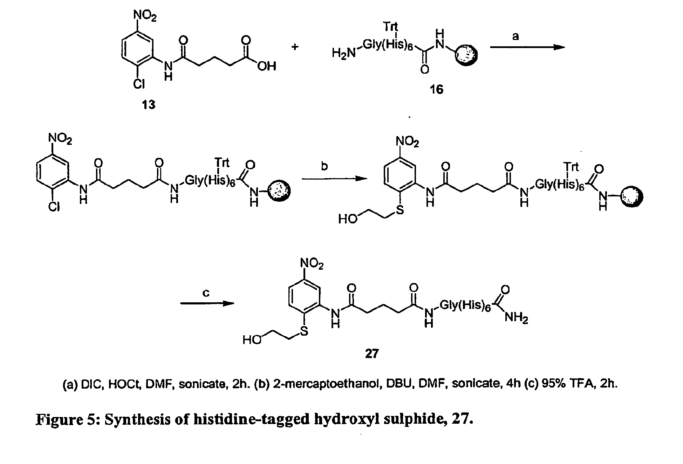

The principle of modified hexa-histidine binding to a column charged with metal ions has been proved from the synthesis of hydroxysulfide, **27** (Figure 5). This purified material (0.2mg) was bound to a Hi-Trap chelation column (Amersham Biosciences) charged with nickel in aqueous buffer (20mM sodium phosphate, 500mM NaCl, pH 6.5), washing the column bound sample the same buffer. Compound elution was afforded by washing the column with a second buffer (20mM sodium phosphate, 500mM NaCl, pH 3.5).

### Example 5 Synthesis of A Hydrophobic Tag

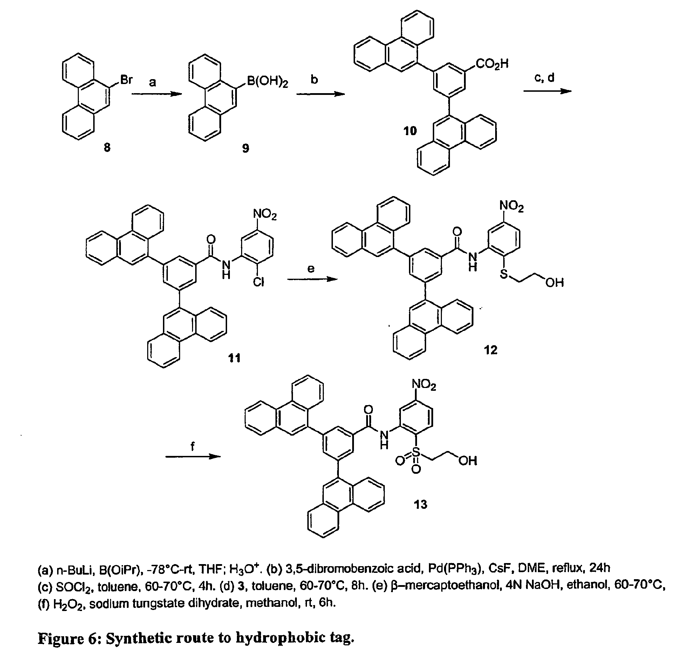

The synthesis of 3,5-(diphenanthren-9-yl)-benzoyl [2-(2-hydroxy-ethanesulfonyl)-5-nitro-phenyl]-anilide [DPBSA] is illustrated in Figure 6. Briefly, the tag, DPBSA is prepared in 5 steps from 9-bromophenanthrene, **27** by first forming the corresponding boronic acid, **28,** and palladium coupling with 3,5-dibromobenzoic acid. The acid chloride of **29** is reacted immediately with 2-chloro-5-nitroaniline, **3,** to form amide, **30.** Sulfanylation is achieved by reaction with β-mercaptoethanol, and selective oxidation to sulfone, **32,** by mild reaction with aqueous hydrogen peroxide.

### Example 6 Purification of a Peptide Using The Hydrophobic Tag DPBSA

DPBSA is reacted with an active material e.g. phosgene, bis(4-nitrophenyl)carbonate), bis(pentafluorophenyl)carbonate or N,N'-di-succinimidyl carbonate, under basic conditions to form a reactive moiety intermediate analogous to that described in Example 2.

The peptide is prepared by standard solid or solution phase synthesis methods with the carboxylic group protected using a conventional protecting group.

The intermediate is then reacted with the peptide in the presence of an amine base (*e.g*. diisopropylethylamine).

The tagged peptide is cleaved from the resin if required using standard cleavage conditions. The crude, tagged peptide is purified by reverse phase HPLC, or absorbed onto solid support as described below.

The crude lyophilised peptide is dissolved in Buffer 1 (8M urea, 0.1M NaH₂PO₄, 0.01M Tris-Cl, pH 8) and agitated with solid support (e.g. polystyrene, or octadecylsilane silica bonded support) The supernatant is removed and the resin is washed with Buffer 2 (8M urea, 0.1M NaH₂PO₄, 0.01M Tris-Cl, pH 6.3).

The supernatant is removed and the protein is cleaved from the captured tag with basic conditions (*e.g*., 20% piperidine in aqueous acetonitrile). Alternatively methods of purification may employ columns packed with a suitable solid support.

### Example 7 Synthesis of an alternative Hydrophobic Tag and Purification Using Such a Tag

The hydrophobic capture moieties, '3-(anthracen-9-yl)-propionyl, Formula IIIA, and 3-(anthracen-9-yl)-2-(anthracen-9-ylmethyl)-propionyl, Formula IIIB have been used as chemical tags for peptides. The hydrophobic nature of the capture moiety has been shown to increase the retention time of substance P on reverse phase HPLC systems. It also imparts unique uv absorption at 365nm, allowing straightforward identification of tagged peptide species.

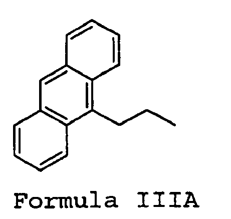

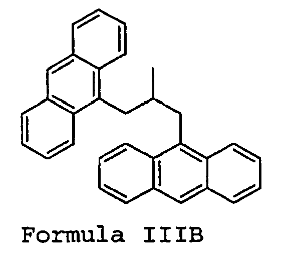

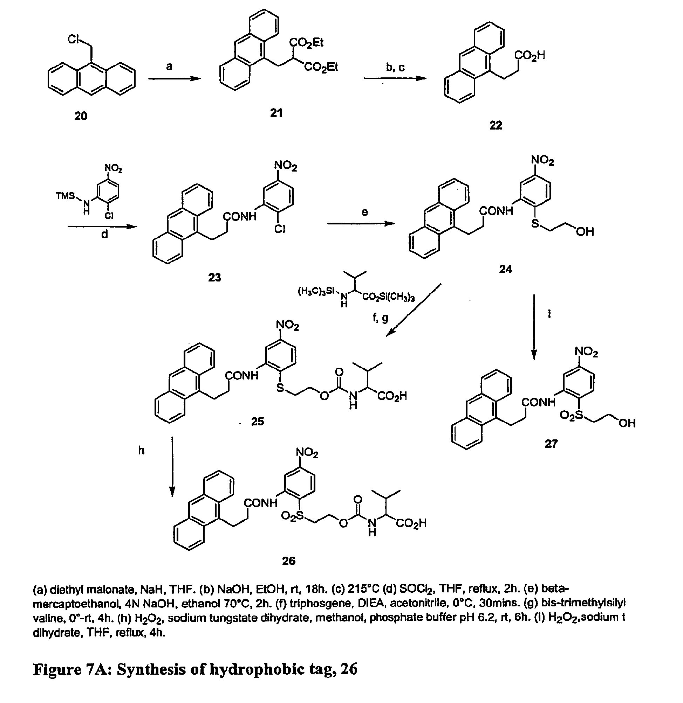

The capture moiety depicted by Formula IIIA has been synthesised by the synthetic route described in Figure 7A. The capture moiety was prepared from 9-chloromethyl anthracene, **20,** by treatment with diethylmalonate, followed by hydrolysis and decarboxylation to form the acid **22.** This was coupled with preformed, silylated 5-chloro-2-nitroaniline, **3,** and sulfanylated by treatment with 2-mercaptoethanol to form structure **24. 24** was oxidised to form sulfone **27.** This can be activated according to an analogous method to that described in Figure 2A.

Alternatively, the free hydroxyl of **24** may be activated by treatment with triphosgene and reaction of the resultant chloroformate with pre-prepared bis-trimethylsilyl valine. Sulphur oxidation under mildly acidic conditions yields the desired tagged amino acid, **26.**

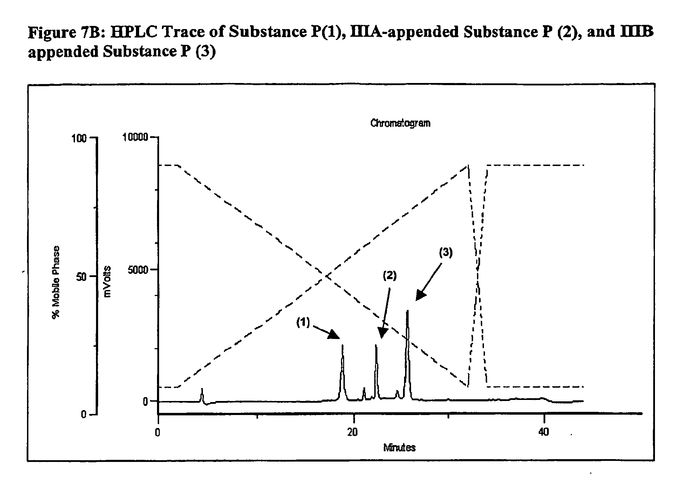

An amino acid, for example valine, bound to a hydrophobic capture agent (*e.g*. 3-anthracen-9-yl-2-anthracen-9-ylmethyl-propionyl) to form tag **26** could be coupled directly with the *N*^{α}-terminus of a resin-bound peptide or protein using standard amide coupling methods (*e.g.* DIC, HOCt, DMF). The resin is then treated with 95% TFA, and the crude peptide purified by preparative HPLC, collecting material that absorbs at 365nm. The hydrophobic capture moiety can be removed from the purified peptide by treatment with base (*e.g*. 20% piperidine in aqueous acetonitrile) and repurified by preparative HPLC. The carboxylic acids of the capture moieties depicted as Formula IIIA and Formula IIIB have independently been coupled to resin-bound Substance P using DIC, HOCt. Cleavage of the resin using 95% TFA formed crude samples of Substance P with no appendage, or appended with IIIA or IIIB via a non-cleavable amide bond. Figure 7B shows an HPLC trace of a mixture of the three compounds described, indicating greatest retention of IIIB appended Substance P.

### Example 8 Synthesis of A Bexa-histidine tagged fluorenyl methanol tag

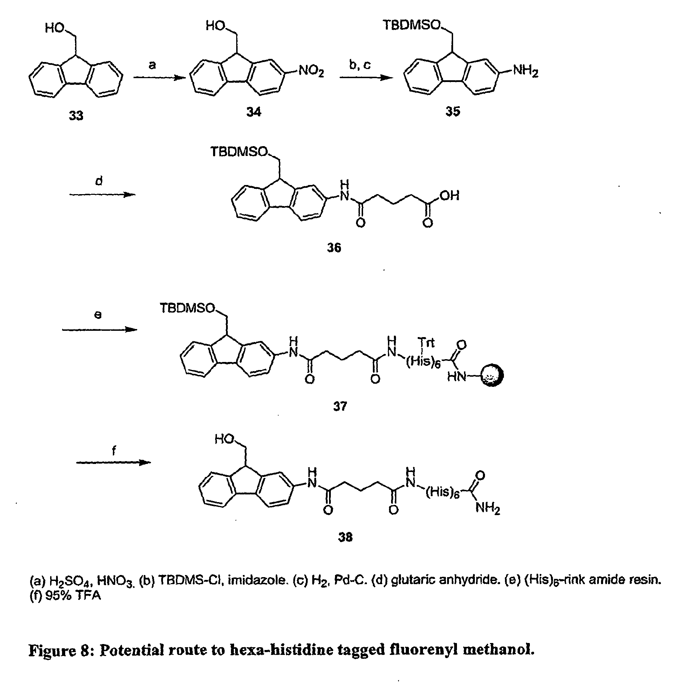

The synthesis of a hexa-histidine tagged fluorenyl methanol tag is shown in Figure 8. Fluorenyl methanol **33,** is nitrated, and the alcohol protected, for example by a silyl group. Nitro reduction forms amine, **35,** which is reacted with glutaric anhydride to form the acid, **36.** This is then coupled with resin-bound hexahistidine (trityl protection on side-chains, and the final tag, **38,** is prepared by treatment with 95% TFA to concomitantly resin and side-chain cleavage.

Various modifications and variations to the described embodiments of the inventions will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

## Claims

1. A tag molecule having formula I
(A)ₙ-C Formula I
wherein A is a capture moiety,
n is at least 1, e.g. 1, 2, 3 or 4; and
C is a peptide binding moiety having formula II 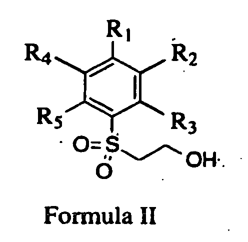 wherein, R₁ and R₃ are each independently H or any C₁₋₆ alkyl group or an electron withdrawing group, R₂ is H or any C₁₋₆ alkyl group, one of R₄ and R₅ is a linker moiety B which links said binding moiety to A; R₄, when not the linker moiety B, is H or any C₁₋₆ alkyl group; R₅, when not the linker moiety B, is an electron withdrawing group or H or any C₁₋₆ alkyl group; wherein at least one of R₁, R₃ and R₅ is an electron withdrawing group;
wherein said capture moiety A is
(i) a metal-binding moiety, or
(ii)a hydrophobic moiety having formula III: 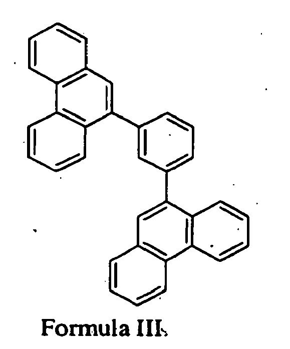
wherein said binding moiety is linked to the linker moiety at position 5 of the central phenyl ring of formula III, and wherein the carbon atoms of the capture moiety may each independently be substituted or unsubstituted; or
(iii) a hydrophobic moiety which comprises the ring structure shown as Formula IIIA, wherein the carbon atoms of the capture moiety may each independently be substituted or unsubstituted :

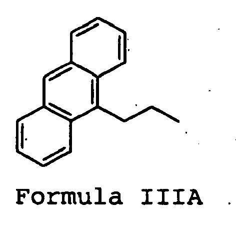

2. The tag molecule according to claim 1, wherein said capture moiety A is (i) a metal-binding moiety.

3. The tag molecule according to claim 2, wherein the metal binding moiety is selected from the group consisting of pyridyl groups, hexa-histidine, iminodiacetic acid (IDA), nitrilotriacetic acid (NTA), 8-hydroxyquinoline and O-phosphoserine.

4. The tag molecule according to claim 3, wherein the metal binding moiety is a pyridyl group.

5. The tag molecule according to claim 4, wherein said capture moiety comprises at least one 2-pyridyl group.

6. The tag molecule according to claim 5 wherein the capture moiety has formula V, wherein the carbon atoms may each independently be substituted or unsubstituted:

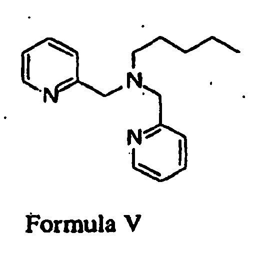

7. The tag molecule according to claim 6 having formula VI

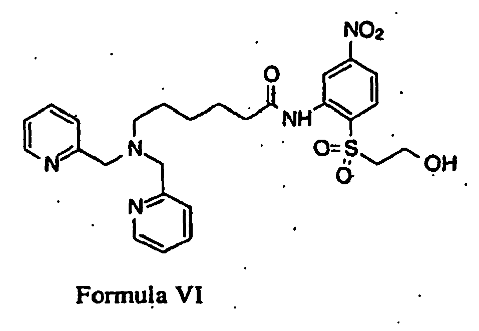

8. The tag molecule according to claim 1 wherein said capture moiety is (ii) a hydrophobic moiety having formula III: 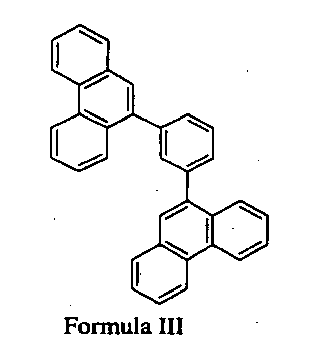 wherein said capture moiety is linked to the linker moiety at position 5 of the central phenyl ring of formula III, and wherein the carbon atoms of the capture moiety may each independently be substituted or unsubstituted.

9. The tag molecule according to claim 8 having formula IV:

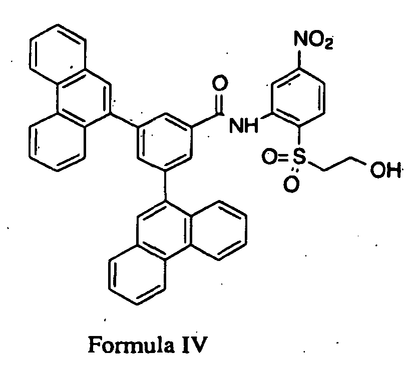

10. The tag molecule according to claim 1 wherein the capture moiety is (iii) a hydrophobic moiety which comprises the ring structure shown as Formula IIIA, wherein the carbon atoms of the capture moiety may each independently be substituted or unsubstituted :

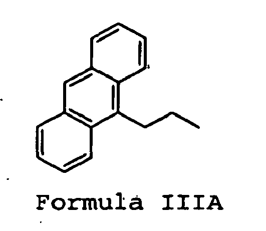

11. The tag molecule according to claim 10 wherein the capture moiety comprises the ring structure shown as Formula IIIB, wherein the carbon atoms of the capture moiety may each independently be substituted or unsubstituted:

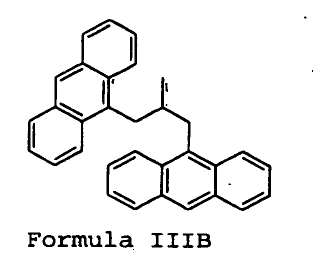

12. The tag molecule according to any one of claims 1 to 11 wherein said electron withdrawing group(s) are selected from the group comprising I, Br, Cl, NO₂, CF₃ and SO₂Me .

13. The tag molecule according to claim 12 wherein said electron withdrawing group(s) is NO₂.

14. The tag molecule according to any one of the preceding claims wherein B comprises an amide, amine, ether, ester, hydrazide, ketone or imine group.

15. A tagged peptide comprising a tag molecule according to any one of the preceding claims attached to a peptide molecule

16. The tagged peptide according to claim 15, wherein said peptide is attached to said tag molecule via a carbamate bond, and has the structure shown as Formula IIB:

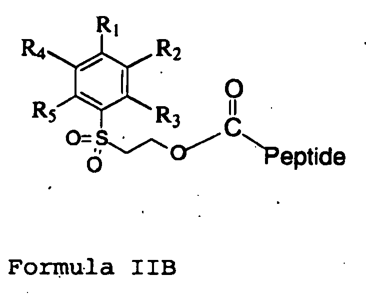

17. A method of tagging a peptide molecule said method comprising the steps of providing a tag molecule according to any one of claims 1 to 14 wherein the hydroxy group of formula II is
substituted with a reactive moiety, and reacting the substituted tag molecule with a peptide molecule
wherein said reactive moiety is a moiety having formula VII: 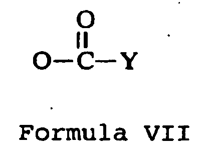 wherein Y is any halogen, preferably Cl, or said reactive moiety comprises a carbonyldioxy moiety.

18. A method for the modification of peptides for facilitating purification thereof, comprising the step of: attaching a tag molecule according to any one of claims 1 to 14 at the end of a peptide chain
during synthesis thereof.

19. A tagged amino acid comprising a tag molecule according to any one of claims 1 to 14 attached to an amine acid

20. The tagged amino acid according to claim 19, wherein said amino acid is attached to said tag molecule via a carbamate bond, and has structure shown as Formula IIC:

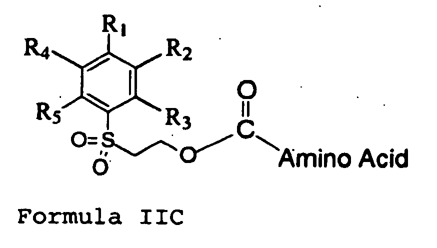

21. A method of tagging an amino acid comprising the steps of
a) providing a tag molecule according any one of claims 1 to 14 wherein the hydroxy group of Formula II is substituted with a reactive moiety,
wherein said reactive moiety is a moiety having formula VII: 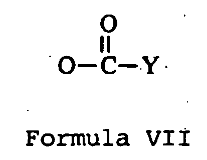 wherein Y is any halogen, preferably Cl,
or said reactive moiety comprises a carbonyldioxy moiety, nd
b) reacting the substituted tag molecule with the N-terminus of the amino acid.

22. The method according to claim 21 further comprising the step
c) coupling the amino acid to a further amino acid or peptide molecule

23. The method according to claim 17, 21 or 22 wherein said substituted tag molecule is formed by reacting the tag molecule with phosgene, bis(4-nitrophenyl)carbonate, bis(pentafluorophenyl) carbonate or N,N'-di-succinimidyl carbonate under suitable conditions.

24. A method for the modification of a peptide for facilitating purification thereof, comprising the steps:
a) attaching a tag molecule according to any one of claims 1 to 14 to the N-terminus.of an amino acid, and
b) coupling the tagged amino acid formed in step (a) to the peptide molecule.

25. A method of purifying a peptide comprising:
a) providing a sample comprising a tagged peptide according to claim 15 or 16 or prepared according to the method of any one of claims 17, 18, 22,or 24.
b) bringing said sample into contact with a capture receptor with which the capture moiety has affinity,
c) removing unbound molecules, for example by washing
d) optionally removing bound tagged peptide from the capture receptor, and
e) cleaving the peptide from the binding moiety of the tag molecule.

26. A method of spatially orientating peptides on a surface in a substantially uniform direction comprising the steps of:
a) providing tagged peptides according to claim 15 or 16 or prepared according to the method of any one of claims 17, 18, 22,or 24,
b) bringing the tagged molecules of step a) into contact with a capture receptor with which the capture moiety has affinity,
c) allowing the tagged molecules to bind to the surface via interaction of the capture moiety with the capture receptor.

27. A diagnostic kit for the detection or purification of a peptide, said kit comprising:
a) a molecular tag according to any one of claims 1 to 14 or a tagged amino acid according to claim 19 or 20, and
b) a capture receptor which can bind the capture moiety of the molecular tag.

28. A tag molecule having formula I
(A)ₙ-C Formula I
wherein A is a capture moiety,
n is at least 1, e.g. 1, 2, 3 or 4; and
C is a peptide binding moiety having formula IID 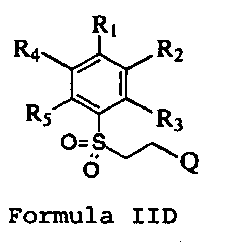
wherein Q is:
(a) a moiety having formula VII: 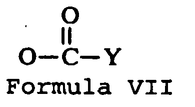 wherein Y is a halogen;
or
(b) a reactive moiety comprising a carbonyldioxy moiety;
and wherein, R₁ and R₃ are each independently H or any C₁₋₆ alkyl group or an electron withdrawing group, R₂ is H or any C₁₋₆ alkyl group, one of R₄ and R₅ is a linker moiety B which links said binding moiety to A; R₄, when not the linker moiety B, is H or any C₁₋₆ alkyl group; R₅, when not the linker moiety B, is an electron withdrawing group or H or any C₁₋₆ alkyl group; wherein at least one of R₁, R₃ and R₅ is an electron withdrawing group;
wherein said capture moiety A is
(i) a metal-binding moiety, or
(ii)a hydrophobic moiety having formula III: 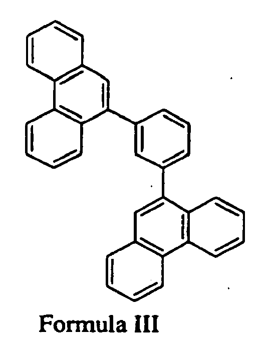
wherein said binding moiety is linked to the linker moiety at position 5 of the central phenyl ring of formula III, and wherein the carbon atoms of the capture moiety may each independently be substituted or unsubstituted; or
(iii) a hydrophobic moiety which comprises the ring structure shown as Formula IIIA, wherein the carbon atoms of the capture moiety may each independently be substituted or unsubstituted :

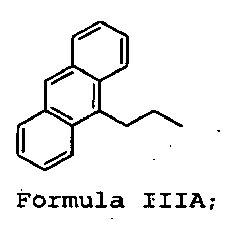

## Patentansprüche

1. Ein Markierungsmolekül mit der Formel I:
(A)ₙ-C Formel I,
wobei A ein Fangteil ist,
n mindestens 1, z. B. 1, 2, 3 oder 4, ist und
C ein Peptid bindender Teil mit der Formel II ist: 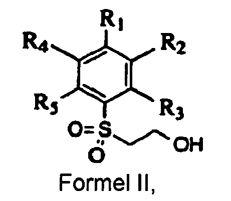 wobei R₁ und R₃ jeweils unabhängig H oder eine beliebige C₁₋₆-Alkylgruppe oder eine elektronenziehende Gruppe sind, R₂ H oder eine beliebige C₁₋₆-Alkylgruppe ist, einer von R₄ und R₅ ein Linker-Teil B ist, der den bindenden Teil mit A verknüpft; R₄, wenn er nicht der Linker-Teil B ist, H oder eine beliebige C₁₋₆-Alkylgruppe ist; R₅, wenn er nicht der Linker-Teil B ist, eine elektronenziehende Gruppe oder H oder eine beliebige C₁₋₆-Alkylgruppe ist; wobei mindestens einer von R₁, R₃ und R₅ eine elektronenziehende Gruppe ist;
wobei der Fangteil A Folgendes ist:
(i) ein Metall bindender Teil oder
(ii) ein hydrophober Teil mit der Formel III: 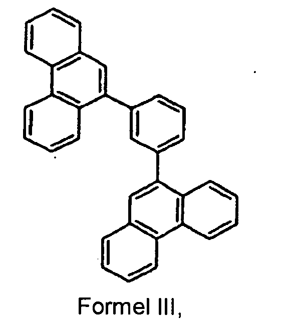 wobei der bindende Teil mit dem Linker-Teil an Position 5 des mittleren Phenylrings von Formel III verknüpft ist und wobei die Kohlenstoffatome des Fangteils jeweils unabhängig substituiert oder nicht substituiert sein können; oder
(iii) ein hydrophober Teil, der die als Formel IIIA gezeigte Ringstruktur beinhaltet, wobei die Kohlenstoffatome des Fangteils jeweils unabhängig substituiert oder nicht substituiert sein können:

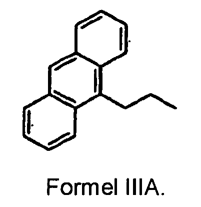

2. Markierungsmolekül gemäß Anspruch 1, wobei der Fangteil A (i) ein Metall bindender Teil ist.

3. Markierungsmolekül gemäß Anspruch 2, wobei der Metall bindende Teil aus der Gruppe, bestehend aus Pyridylgruppen, Hexahistidin, lminodiessigsäure (IDA), Nitrilotriessigsäure (NTA), 8-Hydroxychinolin and O-Phosphoserin, ausgewählt ist.

4. Markierungsmolekül gemäß Anspruch 3, wobei der Metall bindende Teil eine Pyridylgruppe ist.

5. Markierungsmolekül gemäß Anspruch 4, wobei der Fangteil mindestens eine 2-Pyridylgruppe beinhaltet.

6. Markierungsmolekül gemäß Anspruch 5, wobei der Fangteil die Formel V aufweist, wobei die Kohlenstoffatome jeweils unabhängig substituiert oder nicht substituiert sein können:

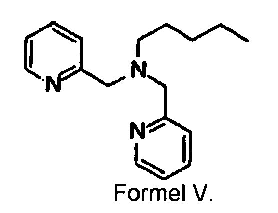

7. Markierungsmolekül gemäß Anspruch 6 mit der Formel VI:

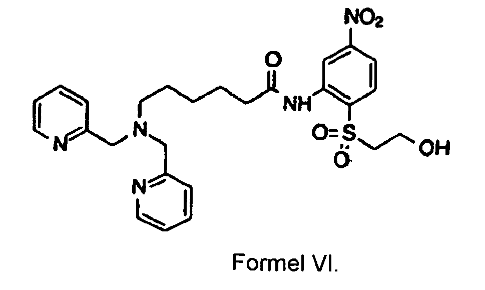

8. Markierungsmolekül gemäß Anspruch 1, wobei der Fangteil (ii) ein hydrophober Teil mit der Formel III ist: 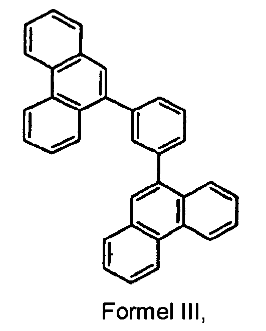 wobei der Fangteil mit dem Linker-Teil an Position 5 des mittleren Phenylrings von Formel III verknüpft ist und wobei die Kohlenstoffatome des Fangteils jeweils unabhängig substituiert oder nicht substituiert sein können.

9. Markierungsmolekül gemäß Anspruch 8 mit der Formel IV:

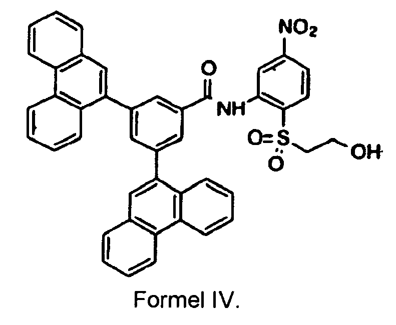

10. Markierungsmolekül gemäß Anspruch 1, wobei der Fangteil (iii) ein hydrophober Teil ist, der die als Formel IIIA gezeigte Ringstruktur beinhaltet, wobei die Kohlenstoffatome des Fangteils jeweils unabhängig substituiert oder nicht substituiert sein können:

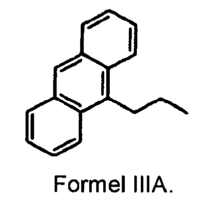

11. Markierungsmolekül gemäß Anspruch 10, wobei der Fangteil die als Formel IIIB gezeigte Ringstruktur beinhaltet, wobei die Kohlenstoffatome des Fangteils jeweils unabhängig substituiert oder nicht substituiert sein können:

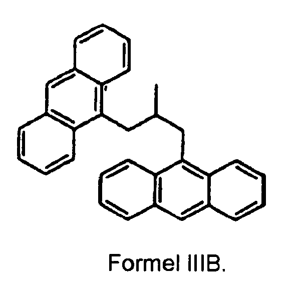

12. Markierungsmolekül gemäß einem der Ansprüche 1 bis 11, wobei die elektronenziehende(n) Gruppe(n) aus der Gruppe, die 1, Br, Cl, NO₂, CF₃ und SO₂Me beinhaltet, ausgewählt ist/sind.

13. Markierungsmolekül gemäß Anspruch 12, wobei die elektronenziehende(n) Gruppe(n) NO₂ ist/sind.

14. Markierungsmolekül gemäß einem der vorhergehenden Ansprüche, wobei B eine Amid-, Amin-, Ether-, Ester-, Hydrazid-, Keton- oder Imingruppe beinhaltet.

15. Ein markiertes Peptid, das ein Markierungsmolekül gemäß einem der vorhergehenden Ansprüche beinhaltet, welches an einem Peptidmolekül angebracht ist.

16. Markiertes Peptid gemäß Anspruch 15, wobei das Peptid über eine Carbamatbindung an dem Markierungsmolekül angebracht ist und die als Formel IIB gezeigte Struktur aufweist:

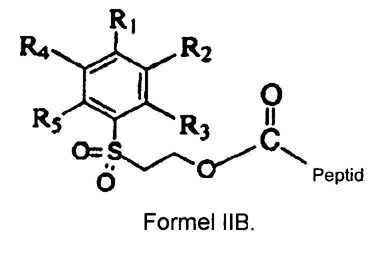

17. Ein Verfahren zum Markieren eines Peptidmoleküls, wobei das Verfahren die Schritte des Bereitstellens eines Markierungsmoleküls gemäß einem der Ansprüche 1 bis 14, wobei die Hydroxygruppe von Formel 11 mit einem reaktiven Teil substituiert ist, und des Reagierenlassens des substituierten Markierungsmoleküls mit einem Peptidmolekül beinhaltet, wobei der reaktive Teil ein Teil mit der Formel VII ist: 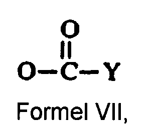 wobei Y ein beliebiges Halogen, vorzugsweise Cl, ist,
oder der reaktive Teil einen Carbonyldioxyteil beinhaltet.

18. Ein Verfahren für die Modifizierung von Peptiden zur Erleichterung ihrer Reinigung, das den folgenden Schritt beinhaltet: Anbringen eines Markierungsmoleküls gemäß einem der Ansprüche 1 bis 14 am Ende einer Peptidkette während ihrer Synthese.

19. Eine markierte Aminosäure, die ein Markierungsmolekül gemäß einem der Ansprüche 1 bis 14 beinhaltet, das an einer Aminosäure angebracht ist.

20. Markierte Aminosäure gemäß Anspruch 19, wobei die Aminosäure über eine Carbamatbindung an dem Markierungsmolekül angebracht ist, und die die als Formel IIC gezeigte Struktur aufweist:

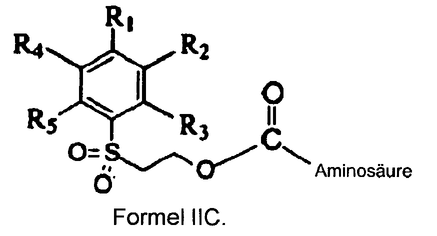

21. Ein Verfahren zum Markieren einer Aminosäure, das die folgenden Schritte beinhaltet:
a) Bereitstellen eines Markierungsmoleküls gemäß einem der Ansprüche 1 bis 14, wobei die Hydroxygruppe von Formel II kit einem reaktiven Teil substituiert ist,
wobei der reaktive Teil ein Teil mit der Formel VII ist: 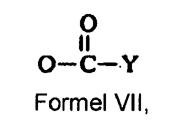 wobei Y ein beliebiges Halogen, vorzugsweise Cl, ist,
oder der reaktive Teil einen Carbonyldioxyteil beinhaltet, und
b) Reagierenlassen des substituierten Markierungsmoleküls mit dem N-Terminus der Aminosäure.

22. Verfahren gemäß Anspruch 21, das ferner den folgenden Schritt beinhaltet:
c) Koppeln der Aminosäure an eine weitere Aminosäure oder ein Peptidmolekül.

23. Verfahren gemäß Anspruch 17, 21 oder 22, wobei das substituierte Markierungsmolekül gebildet wird, indem das Markierungsmolekül unter geeigneten Bedingungen mit Phosgen, Bis(4-nitrophenyl)carbonat, Bis(pentafluorphenyl)carbonat oder N,N'-Di-succinimidyl-carbonat reagieren gelassen wird.

24. Ein Verfahren für die Modifizierung eines Peptids zur Erleichterung seiner Reinigung, das die folgenden Schritte beinhaltet:
a) Anbringen eines Markierungsmoleküls gemäß einem der Ansprüche 1 bis 14 am N-Terminus einer Aminosäure und
b) Koppeln der in Schritt (a) gebildeten markierten Aminosäure an das Peptidmolekül.

25. Ein Verfahren zum Reinigen eines Peptids, das Folgendes beinhaltet:
a) Bereitstellen einer Probe, die ein markiertes Peptid gemäß Anspruch 15 oder 16 oder eines, das gemäß dem Verfahren aus einem der Ansprüche 17, 18, 22 oder 24 zubereitet ist, beinhaltet,
b) In-Kontakt-Bringen der Probe mit einem Fangrezeptor, zu dem der Fangteil eine Affinität aufweist,
c) Entfernen nicht gebundener Moleküle, beispielsweise durch Waschen,
d) optionales Entfernen von gebundenem markiertem Peptid von dem Fangrezeptor und
e) Spalten des Peptids von dem bindenden Teil des Markierungsmoleküls.

26. Ein Verfahren zum räumlichen Ausrichten von Peptiden auf einer Oberfläche in einer im Wesentlichen einheitlichen Richtung, das die folgenden Schritte beinhaltet:
a) Bereitstellen markierter Peptide gemäß Anspruch 15 oder 16 oder solcher, die gemäß dem Verfahren aus einem der Ansprüche 17, 18, 22 oder 24 zubereitet sind,
b) In-Kontakt-Bringen der markierten Moleküle aus Schritt a) mit einem Fangrezeptor, zu dem der Fangteil eine Affinität aufweist,
c) Zulassen, dass die markierten Moleküle über die Wechselwirkung zwischen dem Fangteil und dem Fangrezeptor an die Oberfläche binden.

27. Eine diagnostische Ausstattung zur Erkennung oder Reinigung eines Peptids, wobei die Ausstattung Folgendes beinhaltet:
a) eine molekulare Markierung gemäß einem der Ansprüche 1 bis 14 oder eine markierte Aminosäure gemäß Anspruch 19 oder 20 und
b) einen Fangrezeptor, der den Fangteil der molekularen Markierung binden kann.

28. Ein Markierungsmolekül mit der Formel I:
(A)ₙ-C Formel I,
wobei A ein Fangteil ist,
n mindestens 1, z. B. 1, 2, 3 oder 4, ist und
C ein Peptid bindender Teil mit der Formel IID ist: 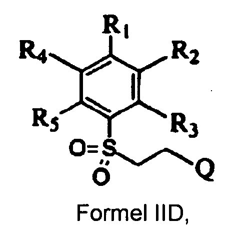 wobei Q Folgendes ist:
(a) ein Teil mit der Formel VII: 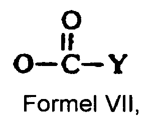 wobei Y ein Halogen ist;
oder
(b) ein reaktiver Teil, der einen Carbonyldioxyteil beinhaltet;
und wobei R₁ und R₃ jeweils unabhängig H oder eine beliebige C₁₋₆-Alkylgruppe oder eine elektronenziehende Gruppe sind, R₂ H oder eine beliebige C₁₋₆-Alkylgruppe ist, einer von R₄ und R₅ ein Linker-Teil B ist, der den bindenden Teil mit A verknüpft; R₄, wenn er nicht der Linker-Teil B ist, H oder eine beliebige C₁₋₆-Alkylgruppe ist; R₅, wenn er nicht der Linker-Teil B ist, eine elektronenziehende Gruppe oder H oder eine beliebige C₁₋₆-Alkylgruppe ist; wobei mindestens einer von R₁, R₃ und R₅ eine elektronenziehende Gruppe ist;
wobei der Fangteil A Folgendes ist:
(i) ein Metall bindender Teil oder
(ii) ein hydrophober Teil mit der Formel III: 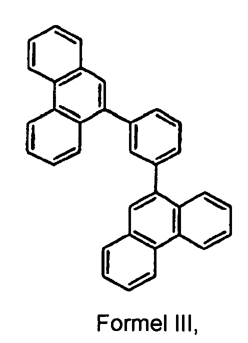 wobei der bindende Teil mit dem Linker-Teil an Position 5 des mittleren Phenylrings von Formel III verknüpft ist und wobei die Kohlenstoffatome des Fangteils jeweils unabhängig substituiert oder nicht substituiert sein können; oder
(iii) ein hydrophober Teil, der die als Formel IIIA gezeigte Ringstruktur beinhaltet, wobei die Kohlenstoffatome des Fangteils jeweils unabhängig substituiert oder nicht substituiert sein können:

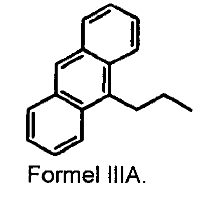

## Revendications

1. Une molécule marqueur ayant la formule 1
(A)ₙ-C Formule I
où A est un fragment de capture,
n est au moins 1, par ex. 1, 2, 3 ou 4 ; et
C est un fragment de liaison de peptide ayant la formule II 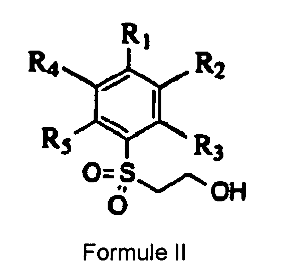 où, R₁ et R₃ sont chacun indépendamment H ou n'importe quel groupe alkyle C₁₋₆ ou un groupe de retrait d'électron, R₂ est H ou n'importe quel groupe alkyle C₁₋₆, soit R₄, soit R₅ est un fragment lieur B qui lie ledit fragment de liaison à A ; R₄, lorsqu'il n'est pas le fragment lieur B, est H ou n'importe quel groupe alkyle C₁₋₆ ; R₅, lorsqu'il n'est pas le fragment lieur B, est un groupe de retrait d'électron ou H ou n'importe quel groupe alkyle C₁₋₆ où au moins soit R₁, soit R₃, soit R₅ est un groupe de retrait d'électron ;
où ledit fragment de capture A est
(i) un fragment de liaison de métal, ou
(ii) un fragment hydrophobe ayant la formule III: 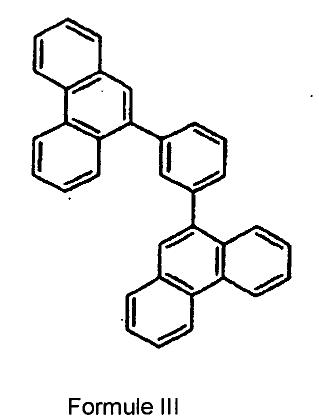
où ledit fragment de liaison est lié au fragment lieur à la position 5 du cycle phényle central de la formule III, et où les atomes de carbone du fragment de capture peuvent être chacun indépendamment substitués ou non substitués ; ou
(iii) un fragment hydrophobe qui comprend la structure cyclique montrée sous forme de Formule IIIA, où les atomes de carbone du fragment de capture peuvent être chacun indépendamment substitués ou non substitués :

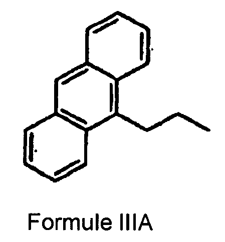

2. La molécule marqueur selon la revendication 1, où ledit fragment de capture A est (i) un fragment de liaison de métal.

3. La molécule marqueur selon la revendication 2, où le fragment de liaison de métal est sélectionné dans le groupe consistant en groupes pyridyle, hexa-histidine, acide iminodiacétique (IDA), acide nitrilotriacétique (NTA), 8-hydroxyquinoline et O-phosphosérine.

4. La molécule marqueur selon la revendication 3, où le fragment de liaison de métal est un groupe pyridyle.

5. La molécule marqueur selon la revendication 4, où ledit fragment de capture comprend au moins un groupe 2-pyridyle.

6. La molécule marqueur selon la revendication 5 où le fragment de capture a la formule V, où les atomes de carbone peuvent être chacun indépendamment substitués ou non substitués :

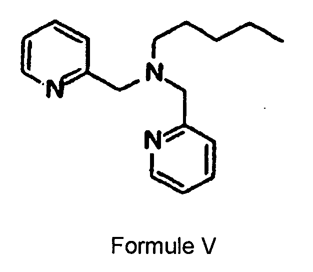

7. La molécule marqueur selon la revendication 6 ayant la formule VI

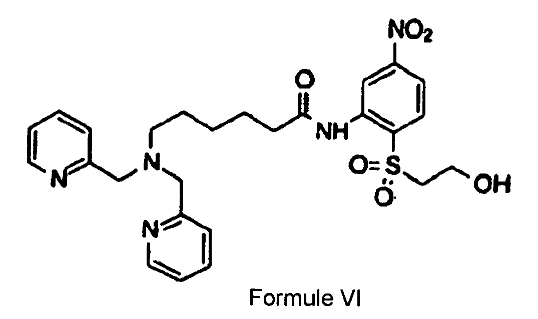

8. La molécule marqueur selon la revendication 1 où ledit fragment de capture est (ii) un fragment hydrophobe ayant la formule III : 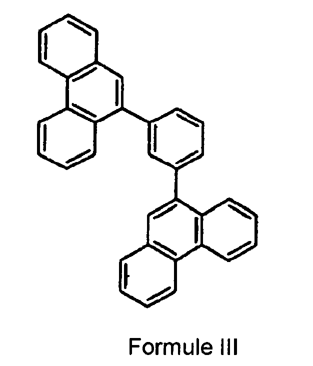 où ledit fragment de capture est lié au fragment lieur à la position 5 du cycle phényle central de formule III, et où les atomes de carbone du fragment de capture peuvent être chacun indépendamment substitués ou non substitués.

9. La molécule marqueur selon la revendication 8 ayant la formule IV :

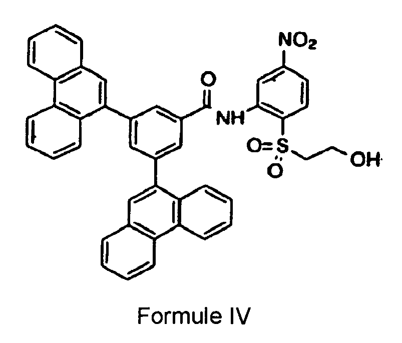

10. La molécule marqueur selon la revendication 1 où le fragment de capture est (iii) un fragment hydrophobe qui comprend la structure cyclique montrée sous forme de Formule IIIA, où les atomes de carbone du fragment de capture peuvent être chacun indépendamment substitués ou non substitués :

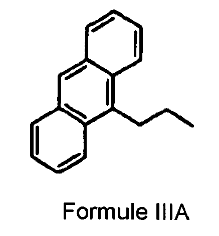

11. La molécule marqueur selon la revendication 10 où le fragment de capture comprend la structure cyclique montrée sous forme de Formule IIIB, où les atomes de carbone du fragment de capture peuvent être chacun indépendamment substitués ou non substitués :

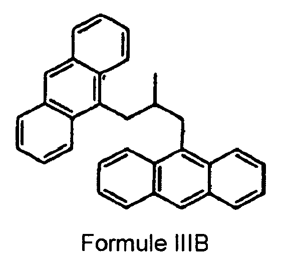

12. La molécule marqueur selon n'importe laquelle des revendications 1 à 11 où ledit (lesdits) groupe(s) de retrait d'électron sont sélectionnés dans le groupe comprenant 1, Br, Cl, NO₂ CF₃ et SO₂Me.

13. La molécule marqueur selon la revendication 12 où ledit (lesdits) groupe(s) de retrait d'électron est (sont) NO₂.

14. La molécule marqueur selon n'importe laquelle des revendications précédentes où B comprend un groupe amide, amine, éther, ester, hydrazide, cétone ou imine.

15. Un peptide marqué comprenant une molécule marqueur selon n'importe laquelle des revendications précédentes attachée à une molécule peptidique.

16. Le peptide marqué selon la revendication 15, où ledit peptide est attaché à ladite molécule marqueur via une liaison carbamate, et a la structure montrée sous forme de Formule IIB :

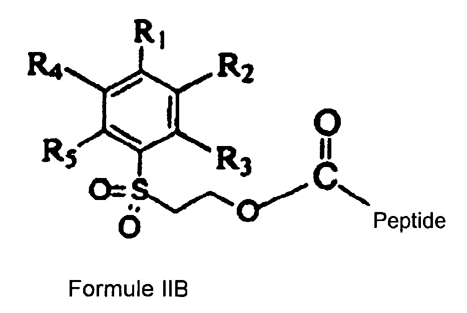

17. Une méthode pour marquer une molécule peptidique, ladite méthode comprenant les étapes de fournir une molécule marqueur selon n'importe laquelle des revendications 1 à 14 où le groupe hydroxy de formule II est substitué par un fragment réactif, et faire réagir la molécule marqueur substituée avec une molécule peptidique où ledit fragment réactif est un fragment ayant la formule VII : 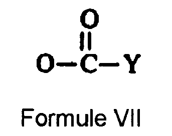 où Y est n'importe quel halogène, de préférence Cl,
ou ledit fragment réactif comprend un fragment carbonyldioxy.

18. Une méthode pour la modification de peptides pour faciliter la purification de ceux-ci, comprenant l'étape : d'attacher une molécule marqueur selon n'importe laquelle des revendications 1 à 14 à l'extrémité d'une chaîne peptidique durant la synthèse de celle-ci.

19. Un acide aminé marqué comprenant une molécule marqueur selon n'importe laquelle des revendications 1 à 14 attachée à un acide aminé.

20. L'acide aminé marqué selon la revendication 19, où ledit acide aminé est attaché à ladite molécule marqueur par le biais d'une liaison carbamate, ayant la structure montrée sous forme de Formule IIC :

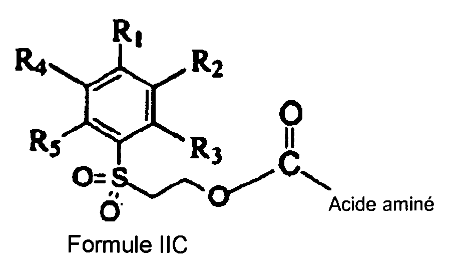

21. Une méthode pour marquer un acide aminé comprenant les étapes de
a) fournir une molécule marqueur selon n'importe laquelle des revendications 1 à 14 où le groupe hydroxy de Formule II est substitué par un fragment réactif, où ledit fragment réactif est un fragment ayant la formule VII : 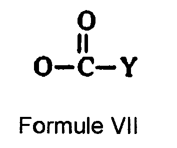 où Y est n'importe quel halogène, de préférence Cl,
ou ledit fragment réactif comprend un fragment carbonyldioxy, et
b) faire réagir la molécule marqueur substituée avec le terminal N de l'acide aminé.

22. La méthode selon la revendication 21 comprenant de plus l'étape c) de coupler l'acide aminé à un acide aminé ou une molécule peptidique autres.

23. La méthode selon la revendication 17, la revendication 21 ou la revendication 22 où ladite molécule marqueur substituée est formée en faisant réagir la molécule marqueur avec du phosgène, bis(4-nitrophényl)carbonate, bis(pentafluorophényl)carbonate ou N,N'-di-succinimidyl carbonate dans des conditions appropriées.

24. Une méthode pour la modification d'un peptide pour faciliter la purification de celui-ci, comprenant les étapes :
a) d'attacher une molécule marqueur selon n'importe laquelle des revendications 1 à 14 au terminal N d'un acide aminé, et
b) de coupler l'acide aminé marqué formé à l'étape (a) à la molécule peptidique.

25. Une méthode pour purifier un peptide comprenant :
a) fournir un échantillon comprenant un peptide marqué selon la revendication 15 ou la revendication 16 ou préparé selon la méthode de n'importe laquelle des revendications 17, 18, 22, ou 24,
b) amener ledit échantillon en contact avec un récepteur de capture avec lequel le fragment de capture possède une affinité,
c) retirer des molécules non liées, par exemple par lavage
d) retirer, facultativement, le peptide marqué lié du récepteur de capture, et
e) cliver le peptide du fragment de liaison de la molécule marqueur.

26. Une méthode pour orienter de façon spatiale des peptides sur une surface dans une direction substantiellement uniforme comprenant les étapes :
a) de fournir des peptides marqués selon la revendication 15 ou la revendication 16 ou préparés selon la méthode de n'importe laquelle des revendications 17, 18, 22, ou 24,
b) d'amener les molécules marquées de l'étape a) en contact avec un récepteur de capture avec lequel le fragment de capture possède une affinité,
c) de permettre aux molécules marquées de se lier à la surface par le biais de l'interaction du fragment de capture avec le récepteur de capture.

27. Un kit de diagnostic pour la détection ou la purification d'un peptide, ledit kit comprenant :
a) un marqueur moléculaire selon n'importe laquelle des revendications 1 à 14 ou un acide aminé marqué selon la revendication 19 ou la revendication 20, et
b) un récepteur de capture qui peut lier le fragment de capture du marqueur moléculaire.

28. Une molécule marqueur ayant la formule I
(A)ₙ-C Formule I
où A est un fragment de capture,
n est au moins 1, par ex. 1, 2, 3 ou 4 ; et
C est un fragment de liaison de peptide ayant la formule IID 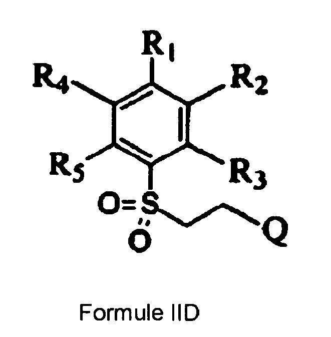 où Q est :
(a) un fragment ayant la formule VII : 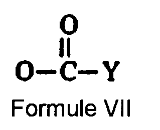
où Y est un halogène ;
ou
(b) un fragment réactif comprenant un fragment carbonyldioxy ;
et où, R₁ et R₃ sont chacun indépendamment H ou n'importe quel groupe alkyle C₁₋₆ ou un groupe de retrait d'électron, R₂ est H ou n'importe quel groupe alkyle C₁₋₆, soit R₄, soit R₅ est un fragment lieur B qui lie ledit fragment de liaison à A ; R₄, lorsqu'il n'est pas le fragment lieur B, est H ou n'importe quel groupe alkyle C₁₋₆ R₅, lorsqu'il n'est pas le fragment lieur B, est un groupe de retrait d'électron ou H ou n'importe quel groupe alkyle C₁₋₆ où au moins soit R₁, soit R₃, soit R₅ est un groupe de retrait d'électron ;
où ledit fragment de capture A est
(i) un fragment de liaison de métal, ou
(ii) un fragment hydrophobe ayant la formule III : 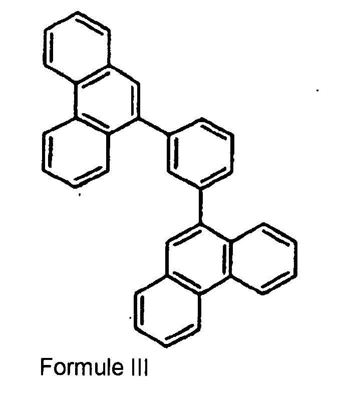
où ledit fragment de liaison est lié au fragment lieur à la position 5 du cycle phényle central de la formule III, et où les atomes de carbone du fragment de capture peuvent être chacun indépendamment substitués ou non substitués ; ou
(iii) un fragment hydrophobe qui comprend la structure cyclique montrée sous forme de Formule IIIA, où les atomes de carbone du fragment de capture peuvent être chacun indépendamment substitués ou non substitués :
